# EUROPÄISCHE PATENTSCHRIFT

(11) **EP 2 123 325 B1**
(45) Veröffentlichungstag und Bekanntmachung des Hinweises auf die Patenterteilung: **07.01.2015**
(21) Anmeldenummer: 09158999.4
(22) Anmeldetag: 29.04.2009
(51) Int. Cl.: A61N 1/375, A61N 1/372, H04B 11/00, H01L 41/107, H04B 13/00, A61N 1/37

(54) **Drahtlose Durchführung für medizinische Implantate**
Wireless execution for medical implants
Passage sans fil pour implants médicaux

(30) Priorität: 23.05.2008 DE 102008024857
(43) Veröffentlichungstag der Anmeldung: 25.11.2009
(73) Patentinhaber: Biotronik CRM Patent AG, 6341 Baar (CH)
(72) Erfinder: Weiss, Ingo, 12435, Berlin (DE); Knorr, Stefan, 10119, Berlin (DE)
(74) Vertreter: Lindner-Vogt, Karin L.

(56) Entgegenhaltungen:
- WO-A1-2008/011570
- US-A1- 2007 293 913
- US-B1- 6 654 638

## Beschreibung

Die Erfindung betrifft ein implantierbares Gerät, insbesondere ein elektromedizinisches Gerät, das in Signalverbindung mit einem implantierbaren Sensor und/oder Aktor steht.

In den letzten Jahrzehnten ist die Heilkunde derart fortgeschritten, dass immer mehr hochentwickelte implantierbare Geräte zur Heilung verschiedener Körperleiden und zur Unterstützung verschiedener Körperfunktionen entwickelt wurden. Viele dieser therapeutischen Geräte werden mit elektrischer Energie betrieben bzw. bringen diese Energie direkt zur Behandlung und/oder Unterstützung von Körperfunktionen in Körpergewebe ein.

Um diese Geräte interaktiv zu gestalten, werden implantierbare Sensoren und/oder Aktoren eingesetzt, die mit dem Gerät in ständiger Verbindung stehen, wie etwa aus der US 6,802,811 B1 bekannt ist. Die Sensoren können zum Beispiel die Herzaktivität oder den Blutfluss überwachen, während die Aktoren bestimmte Körpernerven oder Organe stimulieren.

Viele Implantate sind fernbedienbar, d. h. können von außen aktiviert oder in ihrer Einflussnahme auf den Körper gesteuert bzw. abgefragt werden und können Informationen über den Patientenzustand liefern. Zur Realisierung dieser Fernwirkung (Telemetrie) gibt es eine Reihe etablierter Systeme und einen umfangreichen Bestand an Druckschriften.

Aus der WO 02/056761 ist ein System bekannt, in dem die Informationen sowohl zum Zustand des implantierbaren Geräts selbst als auch zu den Messwerte, die von einem mit dem Gerät verbundenen Sensor erfasst werden, per Ultraschall zu einer externen Überwachungseinheit übermittelt werden. Die akustische Energie kann ferner zur Energieeinspeisung in das implantierbare Gerät genutzt werden. Ein solches System ermöglicht eine kontinuierliche Überwachung des Patienten und vermeidet zusätzliche chirurgische Eingriffe, um zum Beispiel die Batterie des Geräts auszuwechseln. Die Sensoren und/oder Aktoren stellen am Ort des Implantats interaktive Elemente dar, jedoch sind diese nicht fernbedienbar, sondern stehen durch elektrische Leitungen mit dem implantierbaren Gerät in Verbindung.

Üblicherweise umfasst ein implantierbares Gerät ein Gehäuse, welches der Aufnahme von Steuerelektronik, Kondensatoren und Batterien dient. Um die elektrischen Leitungen von den Sensoren und/oder Aktoren mit dem Gerät zu verbinden, wird für jeden Pol ein Draht oder eine andere leitfähige Struktur durch das Gehäuse geführt. Bei den üblichen metallischen Implantatgehäusen muss jeder Draht isoliert durch das Gehäuse geführt werden. Jedoch kommt es zwischen dem Inneren und der Umgebung des Implantats hierbei stets zu einer galvanischen Verbindung.

Aus der DE 10 2006 003 224 A1 ist ein implantierbares Gerät bekannt, welches einen Anschlusskörper umfasst, der an dem Gehäuse befestigt ist und der wenigstens einen, in der Regel aber zwei bis vier, in einer oder mehreren von außen zugänglichen Hohlräumen des Anschlusskörpers angeordneten elektrischen Anschlüsse aufweist, die zum Anschließen einer oder mehrerer Elektrodenleitungen dienen.

Derartige Durchführungen sind kritische Komponenten bezüglich der Dichtheit und der mechanischen Stabilität der Implantate. Grund hierfür ist die mechanische Verbindung zwischen dem Metallgehäuse und der Durchführung, die häufig aus Keramik besteht. Ferner ist der Fertigungsaufwand hoch und die Montage der Durchführungen sehr aufwändig. Eine galvanische Verbindung durch die Gehäusewand hindurch erhöht außerdem die Störanfälligkeit des Implantates gegenüber elektromagnetischen Wechselfeldern, insbesondere dann, wenn außerhalb des Implantats eine lange elektrisch leitende Struktur angeschlossen ist.

Die Dokumente US 6,654,638 B1, US 2007/0293913 A1 sowie WO 2008/011571 A1 offenbaren die Merkmale des Oberbegriffes des Patentanspruchs 1.

Der Erfindung liegt daher die Aufgabe der Bereitstellung einer verbesserten implantierbaren Anordnung mit einem geringeren Fertigungsaufwand zugrunde, in der die Signalübertragung zwischen den innerhalb und außerhalb des Gehäuses angeordneten Komponenten mit hoher Störsicherheit und in einer die Dichtigkeit des Gehäuses auch bei langer Gebrauchsdauer nicht beeinträchtigenden Weise erfolgt.

Diese Aufgabe wird durch eine implantierbare Anordnung mit den Merkmalen des Anspruchs 1 gelöst. Zweckmäßige Fortbildungen des Erfindungsgedankens sind Gegenstand der abhängigen Ansprüche.

Die Erfindung schließt die wesentliche Überlegung ein, die Signalübertragung zwischen den innerhalb und außerhalb des Gehäuses angeordneten Komponenten ohne eine Leitungsverbindung durch die Gehäusewand hindurch zu realisieren. Insgesamt wird erfindungsgemäß eine implantierbare Anordnung vorgeschlagen, die ein implantierbares Gerät mit einer Außenwandung und einen implantierbaren Sensor und/oder Aktor umfasst, welcher in Signalverbindung mit dem implantierbaren Gerät steht, wobei der implantierbare Sensor und/oder Aktor im Gebrauchszustand außerhalb der Außenwandung angeordnet ist und wobei die Signalverbindung zwischen dem Gerät und dem Sensor und/oder Aktor einen elektrischen und einen hierzu in Reihe angeordneten mechanischen Abschnitt hat. Dieser ist von einer Signalübertragung mittels mechanischer Schwingungen durch die geschlossene Außenwandung hindurch gebildet.

Daraus folgt, dass die Signalübertragung potentialfrei erfolgt. Außerdem erfolgt die Übertragung in einem bestimmten Frequenzbereich, während verschiedene Pole frequenzcodiert durch die Außenwandung übertragen werden können. Insbesondere bei metallischen Implantaten ist die Elektronik zuverlässiger gegen elektromagnetische Störungen (EMI), insbesondere starke Wechselfelder wie zum Beispiel bei der Magnetresonanztomographie (MRI), geschützt.

Die Außenwandung kann aus leitfähigem oder isolierendem Material, insbesondere aus Metall, aus Plastik, aus Keramik oder aus beliebigem Material, das für ein Implantat in Frage kommt, bestehen. Hierdurch werden eine geringere mechanische Empfindlichkeit, eine hohe Dichtigkeit des implantierbaren Geräts und ein geringerer Fertigungsaufwand erreicht.

Eine bevorzugte Ausführung der Erfindung besteht darin, dass im mechanischen Abschnitt ein Wandler vorgesehen ist, der aus einem piezoelektrischen Material besteht und der die elektrischen Signale in mechanische Schwingungen oder umgekehrt die mechanischen Schwingungen in elektrische Signale umwandelt. Alternativ kann der mechanische Abschnitt auch einen nicht-piezoelektischen Wandler aufweisen. Beispiel dafür kann eine Magnetspulenanordnung sein, welche die mechanischen Schwingungen in eine Strom- bzw. Spannungsvariation umwandelt oder umkehrt.

Um die mechanischen Schwingungen im Rahmen einer ansonsten insgesamt elektrischen Signalverbindung durch die geschlossene Außenwandung zu übertragen, können jeweils ein piezoelektrischer Wandler auf der einen und der anderen Seite der Außenwandung angeordnet sein. Insbesondere kann mindestens einer der piezoelektrischen Wandler derart starr mit der Außenwandung verbunden sein, dass er die Außenwandungen direkt in Schwingungen versetzt. Grundsätzlich kann bei speziellen Systemen aber die Signalfortleitung auf einer Seite der Geräteaußenwandung auch auf mechanischem Wege erfolgen, so dass die Anordnung nur einen piezoelektrischen Wandler (oder anderen elektromechanischen Wandler) umfasst.

Alternativ hierzu kann, um die mechanischen Schwingungen zu einem weit von der Außenwandung befindlichen Wandler weiterzuleiten, im mechanischen Abschnitt mindestens ein zur Weiterleitung der mechanischen Schwingungen dienliches Element vorgesehen sein. Hierdurch kann die Wandlung an einer von der Außenwandung weit befindlichen Stelle erfolgen, sodass zwischen dieser Stelle und der Außenwandung keine elektrische Verbindung besteht. Vorteilhaft ist, dass sich der Bereich zwischen dieser Stelle und der Außenwandung als unempfindlich gegen elektromagnetische Strahlung (EMI) erweist.

Dieses Element kann aus dielektrischem Material sowie aus jedem geeigneten Material bestehen, das einen wirksamen mechanischen Wellenleiter ergeben kann.

Der piezoelektrische Wandler weist Kontaktflächen auf, an denen elektrische Leitungen zur Einspeisung bzw. Weiterleitung der elektrischen Signale angeordnet sind.

Die Geometrie und die Lage der Kontaktflächen des Wandlers sind wichtige Parameter, die, zusammen mit dem piezoelektrischen Material, die Resonanzfrequenz stark beeinflussen.

Die Kontaktflächen können zum Beispiel sowohl entlang als auch senkrecht zu den Kontaktflächen angeordnet sein.

Um die Verformungsamplitude des Wandlers und damit die Signalamplitude seines Ausgangssignals zu erhöhen, kann dieser beispielsweise aus einer Stapelstruktur bestehen, in der piezoelektrische Schichten und Elektrodenleitungen übereinander abwechseln gestapelt werden.

Die Ansteuerung des Wandlers, der zum Erzeugen der mechanischen Schwingungen genutzt wird, erfolgt zweckmäßigerweise mit einem Schwingkreis, wobei der piezoelektrische Wandler als Kondensator wirkt, der die elektrische Eigenfrequenz des Schwingkreises mindestens mitbestimmt. Um mehrere bzw. diskrete Frequenzen zu erzeugen, kann der Schwingkreis verstimmt werden. Dies erfolgt beispielsweise durch zuschaltbare Kondensatoren.

In einer weiteren Ausführung der Erfindung sind außerhalb der Außenwandung mehrere piezoelektrische Wandler nebeneinander angeordnet, die verschiedene Resonanzfrequenzen besitzen, so dass der innerhalb der Außenwandung befindliche piezoelektrische Wandler mit einer Wechselspannung der Resonanzfrequenz eines oder mehrerer außenliegender Wandler angesteuert wird. Damit kann die gleiche Funktion wie mit einer Anordnung realisiert werden, bei der außerhalb der Außenwandung nur ein einziges piezoelektrisches Element und innerhalb der Außenwandung mehrere piezoelektrische Wandler nebeneinander angeordnet würden.

Es gibt auch die Möglichkeit, Wandlerpaare jeweils entlang der Innen- und Außenwandung zu bilden, die in Resonanz miteinander stehen, wobei sowohl außerhalb als auch innerhalb der Außenwandung mehrere piezoelektrische Wandler mit verschiedenen Resonanzfrequenzen nebeneinander angeordnet sind.

Hierbei kann eine Ausgestaltung vorgesehen sein, in der anstelle der außenliegenden nebeneinander angeordneten Wandler jeweils ein zur Weiterleitung der mechanischen Schwingungen dienliches Element vorgesehen ist, das die Wandlung in elektrischen Signale an einer von der Außenwandung weit entfernten Stelle ermöglicht.

In einer weiteren Ausführung befindet sich auf mindestens einer Seite der Außenwandung mindestens ein piezoelektrischer Wandler, der mit einer ungleichmäßigen Dicke gebildet ist. Hierdurch besitzt ein solcher Wandler verschiedene Eigenfrequenzen, die an verschiedenen Orten auftreten. Ein solcher spezieller Wandler entspricht damit funktional der weiter oben angesprochenen Parallelschaltung mehrerer Wandler mit verschiedenen Resonanzfrequenzen, kann dieser gegenüber aber Platz sparen und gegebenenfalls auch kostengünstiger sein. Um den Frequenzbereich zu erweitern, kann ein mit einer ungleichmäßigen Dicke gekennzeichnete piezoelektrische Wandler sowohl innerhalb als auch außerhalb der Außenwandung angeordnet sein.

Die mechanischen Schwingungen werden in einer wichtigen Ausführung in elektrische Signale umgewandelt und über eine Elektrodenleitung, zu einer Elektrode weitergeleitet, die insbesondere als Stimulations- und/oder Abfühlelektrode zur Stimulation von elektrisch erregbarem Gewebe (Herzmuskel, Gehirnareale, aber auch Hörnerv o. ä.) bzw. zum Erfassen von Gewebspotenzialen ausgeführt ist. Ebenso ist die erfindungsgemäße Art der Signalübertragung aber auch bei Anordnungen einsetzbar, die keine Elektroden, sondern etwa optische Sensoren (mit nachgeschaltetem optoelektronischen Wandler) oder mechanische Aktoren (mit vorgeschaltetem elektromechanischem Wandler) umfassen.

Die Ein/Auskopplung der elektrischen Signale geschieht über genormte Verbindungen, wie zum Beispiel IS-1 oder IS-4 bzw. andere zukünftige, standardisierte Steckverbindungen, oder ein eigenständiges Steckersystem.

Um die elektrischen Signale bzw. die Wechselspannung gleichrichten zu können, kann der elektrische Abschnitt zusätzlich einen Gleichrichter aufweisen.

Um den an der Außenwandung befindlichen mechanischen Abschnitt der Signalübertragung zu schützen bzw. stabiler zu gestalten, ist bevorzugt eine Abdeckung außerhalb der Außenwandung angebracht. Die Elektrodenleitung ist dann ggf. an dieser Abdeckung angeschlossen, wobei die Leitungsenden aus der Abdeckung herausragen, und mit den Sensoren und/oder Aktoren in Signalverbindung stehen.

In einer besonders zweckmäßigen Ausgestaltung hiervon, kann die Abdeckung zwei Anschlüsse aufweisen, die mit der Elektrodenleitung und den außerhalb der Außenwandung befindlichen piezoelektrischen Wandlern in Verbindung stehen, um zusätzlich zu den aus der Abdeckung herausragenden Elektrodenenden weitere Anschlussmöglichkeiten für die Sensoren und/oder Aktoren bereitzustellen.

In einer weiteren Ausführung dieser Erfindung ist eine wechselbare Elektrodenanschlussstruktur vorgesehen, die direkt außerhalb an der Außenwandung angebracht werden kann. Hierbei weist die wechselbare Struktur ein Elektrodengehäuse und eine an dem Gehäuse fixierte Elektrodenleitung auf. Die Struktur schließt mindestens einen piezoelektrischen Wandler zur Wandlung der mechanischen Schwingungen in elektrische Signale ein, wobei die Elektrodenleitungsenden aus dem Elektrodengehäuse herausragen. Die mechanische Befestigung dieser wechselbaren Struktur kann mit verschiedenen Methoden geschehen. Möglich sind z.B. eine Art Bajonett-Verschluss, ein Schraubanschluss sowie eine Klemmbefestigung. Vorteil einer solchen Anordnung ist die Möglichkeit, bei Bedarf nur einen Teil der implantierbaren Anordnung auszuwechseln, was Kostenersparnisse erbringt.

Die vorliegende Erfindung kann in verschiedenen Bereichen Anwendung finden, wie beispielsweise auf dem Gebiet der Kardiologie, der Hals-Nasen-Ohren-Heilkunde, oder der Orthopädie. In der Kardiologie kann das implantierbare Gerät ein Herzstimulator, der implantierbare Sensor ein Mittel zur Messung der Herzkontraktionen und der implantierbare Aktor ein Mittel zur Abgabe von Stimulierungsimpulsen darstellen, während in der Hals-Nasen-Ohren-Heilkunde das implantierbare Gerät als Hörgerätimplantat und der Aktor als Mittel zur Stimulierung des Hörnervs Anwendung finden kann. Auch in der Orthopädie sind verschiedene Einsatzmöglichkeiten denkbar. So kann das implantierbare Gerät als orthopädisches Implantat sein und als Sensor ein Mittel zur Messung der Körperreize oder einer anderen Größe des Gewebes, wie der Temperatur oder des Drucks, Einsatz finden.

Vorteile und Zweckmäßigkeiten der Erfindung werden im Übrigen deutlich anhand der nachfolgenden Beschreibung einiger ausgewählter Ausführungsbeispiele anhand der Figuren. Von diesen zeigen:
- Fig. 1: eine schematische Darstellung einer implantierbaren Anordnung gemäß der vorliegenden Erfindung;
- Fig. 2: eine schematische Darstellung des mechanischen Abschnittes gemäß einer Ausführungsform der vorliegenden Erfindung;
- Fig. 3: eine schematische Darstellung des mechanischen Abschnittes gemäß einer alternativen Ausführungsform der vorliegenden Erfindung;
- Fig. 4: schematische Darstellungen des mechanischen Abschnittes gemäß verschiedener weiteren Ausführungsformen der vorliegenden Erfindung;
- Fig. 5: eine schematische Darstellung des mechanischen Abschnittes gemäß einer weiteren Ausführungsform der vorliegenden Erfindung;
- Fig. 6: schematische Darstellungen der Weiterleitung der elektrischen Signale gemäß der verschiedenen Ausführungsformen der vorliegenden Erfindung;
- Fig. 7: eine schematische Darstellung der Abdeckung gemäß einer besonderen Ausführungsform der vorliegenden Erfindung,
- Fig. 8: eine schematische Darstellung des Elektrodengehäuses einer wechselbaren Elektrodenstruktur gemäß einer bevorzugten Ausführungsform der vorliegenden Erfindung;
- Fig. 9: eine schematische Darstellung der Befestigung einer Elektrodenanschlussstruktur gemäß einer bevorzugten Ausführungsform der vorliegenden Erfindung.

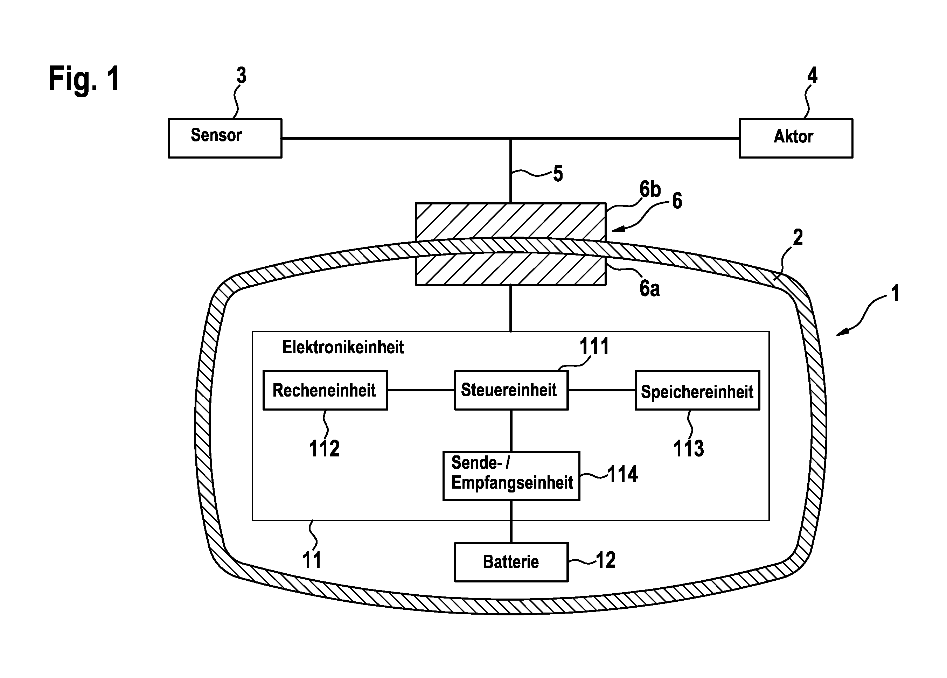

Fig. 1 zeigt ein implantierbares Gerät 1 mit einer Außenwandung 2, die eine rundum geschlossene Oberfläche aus einem einzigen Material besitzt, und zwei implantierbaren elektrischen Komponenten 3 und 4, die im Gebrauchszustand außerhalb der Außenwandung angeordnet sind. Diese Komponenten sind ein Sensor (Messfühler) 3, der bestimmte physikalische oder chemische Eigenschaften eines Gewebes oder Organs erfasst, und ein Aktor 4, der ein Eingangssignal vom Gerät 1 empfängt und hiermit auf den Körper eines Patienten einwirkt. Der implantierbare Sensor 3 und der implantierbare Aktor 4 stehen in Signalverbindung mit dem implantierbaren Gerät 1. Die Signale zwischen dem Gerät 1 und dem Sensor 3 bzw. Aktor 4 werden durch einen elektrischen Abschnitt 5 und einen mechanischen Abschnitt 6 weitergeleitet. Der mechanische Abschnitt 6 ist zum elektrischen Abschnitt 5 in Reihe angeordnet und hat Teilabschnitte 6a und 6b innerhalb und außerhalb der Außenwandung 2.

Innerhalb der Außenwandung 2 enthält das Gerät 1 eine Elektronikeinheit 11, die in Signalverbindung mit dem innerhalb der Außenwandung befindlichen Teil 6a des mechanischen Abschnitts 6 steht. Die Elektronikeinheit 11 enthält eine Steuereinheit 111 zur Betriebssteuerung des Geräts 1 bzw. des Sensors 3 bzw. Aktors 4, eine Rechnereinheit 112 und eine Speichereinheit 113 zum Rechnen und zum Speichern der erfassten Messwerte des Sensor 3 und eine Sende-/Empfangseinheit 114, um mittels einer drahtlosen Kommunikation Daten zu einer (nicht gezeigten) externen Überwachungseinheit zu senden bzw. Steuersignale von einer solchen Einheit zu empfangen. Ferner hat das implantierbare Gerät 1 eine Batterie 12, die als Energiequelle zur Versorgung der elektrischen Elemente der Elektronikeinheit 11 dient. Die Sende-/Empfangseinheit 114 wird dazu benutzt, um die Batterie 12 durch eine drahtlose Energieeinspeisung periodisch aufzuladen.

Der mechanische Abschnitt 6 ermöglicht eine Signalübertragung durch die geschlossene Außenwandung 2 mittels mechanischer Schwingungen. Insbesondere ermöglicht dieser Abschnitt 6 die Übertragung von Stimulationsimpulsen, die von der in Implantatelektronik 11 erzeugt werden, an den Aktor 4 und in ähnlicher Weise der Signale vom Sensor 3 an die Elektronikeinheit 11, ohne eine galvanische Verbindung durch die Außenwandung 2 zu führen. Daraus folgt, dass die Signalübertragung potentialfrei erfolgt. Außerdem erfolgt die Übertragung in einem bestimmten Frequenzbereich, während verschiedene Pole frequenzcodiert durch die Außenwandung übertragen werden können.

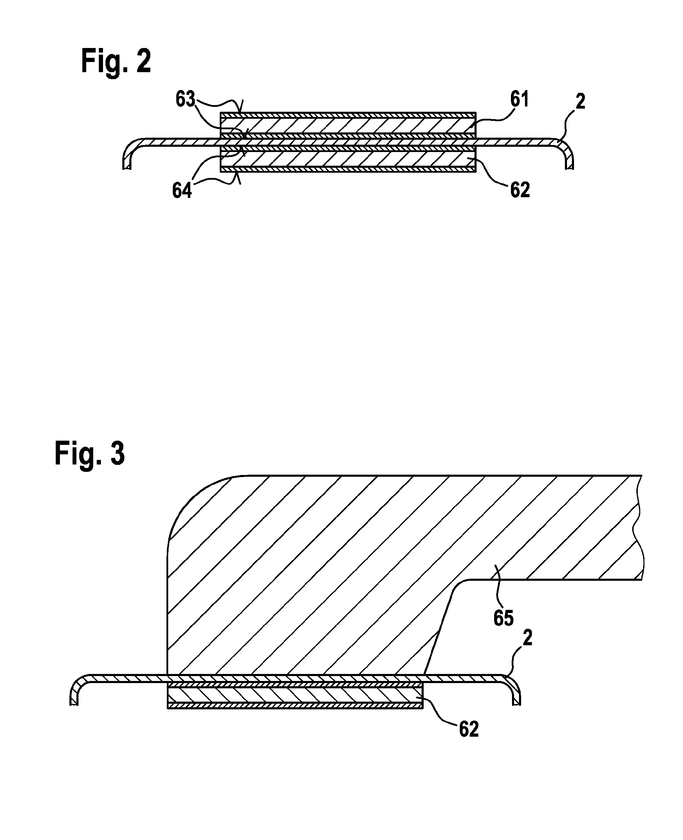

In Fig. 2 wird eine Darstellung des mechanischen Abschnitts 6 gezeigt, in der zwei Wandler, insbesondere piezoelektrische Wandler 61 und 62, welche die elektrischen Signale in mechanische Schwingungen, oder umgekehrt, die mechanischen Schwingungen in elektrische Signale, umwandeln, jeweils auf der einen und der anderen Seite der Außenwandung 2 angeordnet sind. Hierbei sind die piezoelektrischen Wandler 61 und 62 mit der Außenwandung derart starr verbunden, dass sie die Außenwandung 2 direkt in Schwingung versetzen können und der jeweils andere (nicht angesteuerte) Wandler die Schwingung direkt aufnehmen kann.

Jeder Wandler 61 und 62 weist Kontaktflächen 63 und 64 auf, an denen elektrische Leitungen zur Weiterleitung der elektrischen Signale angebracht sind. Die elektrischen Signale werden von der Elektronikeinheit 11 an den innenliegenden Wandler 62 geleitet, wo sie in mechanischen Schwingungen umgewandelt werden. Die mechanischen Schwingungen werden über die Außenwandung 2 an den außenliegenden Wandler 61 übertragen und dort in elektrische Signale umgewandelt. Diese Signale werden schließlich über elektrische Leitungen an den Aktor 4 weitergeleitet. Im umgekehrten Falle, werden die elektrischen Signale von dem Sensor 3 an die innerhalb der Außenwandung 2 befindliche Elektronikeinheit 11 übertragen.

Fig. 3 zeigt eine alternative Ausführung, in der außen an der Außenwandung 2 nicht direkt ein piezoelektrischer Wandler vorgesehen ist, sondern ein (z. B. im Wesentlichen stabförmiges) Element 65, das zur Weiterleitung der mechanischen Schwingungen dient. Die Wandlung in elektrische Signale kann dann an einer anderen Stelle, die sich weit entfernt von der Außenwandung 2 befindet. Dieses Element kann aus dielektrischem Material sowie aus jedem geeigneten Material bestehen, das einen wirksamen mechanischen Wellenleiter ergibt.

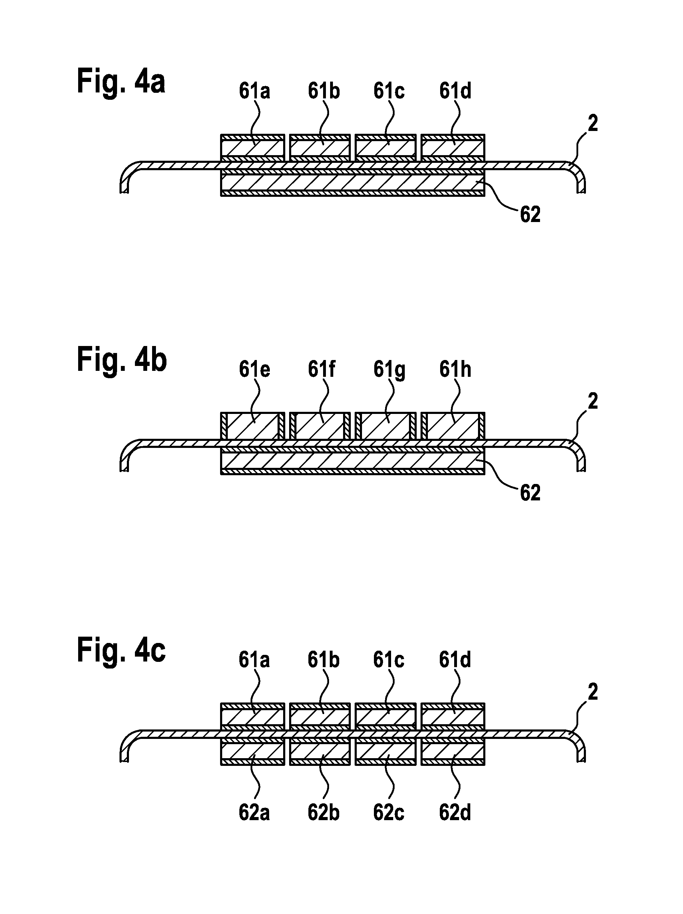

In der Fig. 4a ist eine Ausführung des mechanischen Abschnitts 6 gezeigt, in der außerhalb der Außenwandung 2 mehrere piezoelektrische Wandler 61 a, 61 b, 61 c und 61 d, nebeneinander angeordnet sind. Diese Wandler besitzen verschiedene Resonanzfrequenzen und ermöglichen daher eine Frequenzcodierung. Somit wird der innerhalb der Außenwandung 2 befindliche Wandler 62 mit einer Wechselspannung entsprechend der Resonanzfrequenz eines oder mehrerer außerhalb der Außenwandung 2 positionierten Wandler 61a, 61b, 61c und 61d angesteuert. Der Wandler 62 wird in mechanische Schwingung entsprechend der Frequenz/Frequenzen des/der entsprechenden Wandler 61 a, 61 b, 61 c und 61 d versetzt, die außerhalb der Außenwandung 2 jeweils die resonante mechanische Schwingung erzeugen.

Fig. 4b zeigt eine modifizierte Ausführung, bei der die Kontaktflächen der außenliegenden Wandler 61e, 61f, 61g und 61h nicht entlang der Außenwandung 2, wie in Fig. 4a, sondern senkrecht zur Außenwandung 2 angeordnet sind. Eine Änderung der Kontaktflächenanordnung ermöglicht eine Änderung der Schwingungsrichtung und somit eine Änderung der Resonanzfrequenz der Wandler. Insbesondere erleichtert die in Fig. 4b gezeigte Ausführung die Anordnung der elektrischen Leitungen an den Kontaktflächen und verbessert die Übertragung der mechanischen Schwingungen durch die Außenwandung 2 hindurch.

Fig. 4c zeigt eine weitere Ausführung des mechanischen Abschnitts 6, in der sowohl innerhalb als auch außerhalb der Außenwandung 2 mehrere piezoelektrische Wandler 61a, 61 b, 61c, 61 d und 62a, 62b, 62c, 62d nebeneinander angeordnet sind. Hierbei wird der Wirkungsgrad erhöht, indem jeweils Wandlerpaare, wie zum Beispiel 61a und 62a, in Resonanz zueinander stehen. Außerdem sind die einzelnen Wandler 62a, 62b, 62c und 62d innerhalb der Außenwandung 2 galvanisch getrennt, wodurch sie sich gegenseitig nicht beeinflussen. Sie können sogar die gleiche Frequenz haben, wenn die Kopplung zweier verschiedener Wandlerpaare nicht zu stark ist.

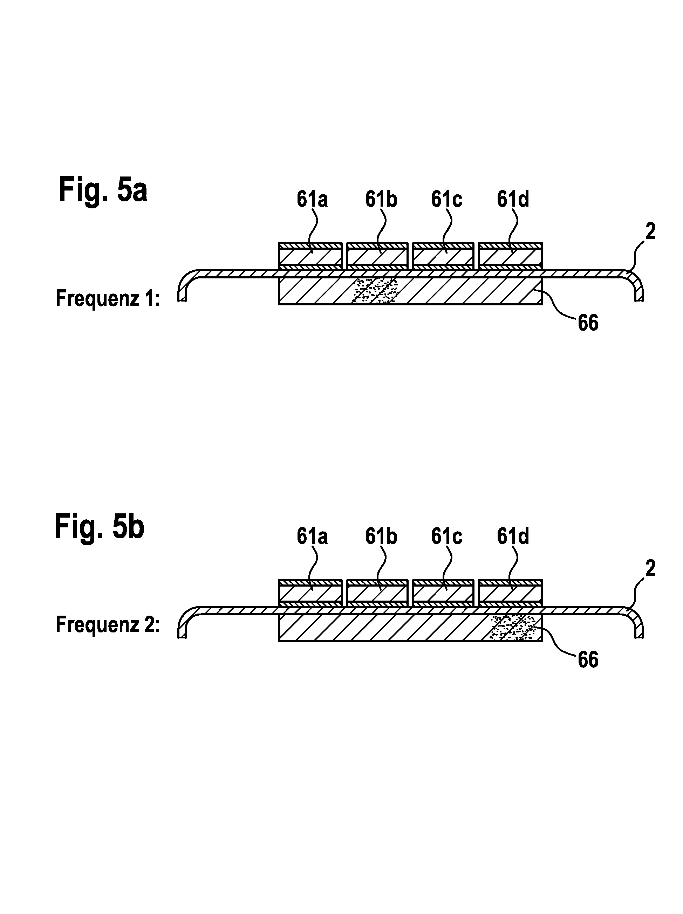

Fig. 5a und 5b stellen eine Ausführung des mechanischen Abschnitts 6 dar, in der innerhalb der Außenwandung 2 ein piezoelektrischer Wandler 66 mit einer ungleichmäßigen Dicke gebildet ist. Ein solcher Wandler besitzt verschiedene Eigenfrequenzen, deren mechanische Schwingungen an verschiedenen Orten des Wandlers auftreten. Wird der Wandler 66 mit einer der Eigenfrequenzen erregt, so schwingt er an der entsprechenden Position besonders stark. Wird auf der anderen Seite der Außenwandung 2 an der entsprechenden Stelle ein piezoelektrischer Wandler mit derselben Eigenfrequenz platziert, so wird primär dieser zum Schwingen angeregt. In Fig. 5a und Fig. 5b entspricht dieser außenliegende Wandler dem piezoelektrischen Wandler 61b bzw. 61d. Hierbei kennzeichnen die dunklen Stellen den frequenzabhängigen Bereich, in dem die Schwingungsamplitude maximal ist.

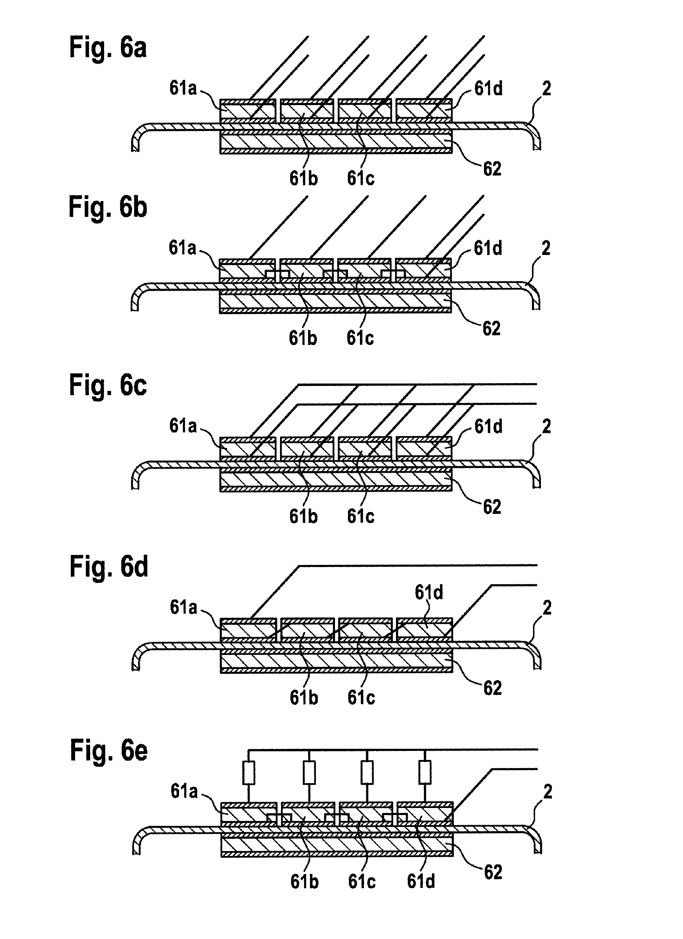

Die Weiterleitung der elektrischen Signale kann auf verschiedene Weise geschehen. Am einfachsten ist es, wenn jeder Pol einzeln mittels zweier elektrischer Leitungen übertragen wird, wie in Fig. 6a dargestellt ist.

Um die Anzahl der elektrischen Leitungen zu reduzieren, können die Bezugspotentiale verbunden werden, wie in Fig. 6b gezeigt ist. Die Anzahl der Leitungen kann weiter reduziert werden, wenn die verschiedenen Signale bei verschiedenen Frequenzen übertragen werden. Verschiedene Frequenzen können auf derselben Leitung übertragen werden, ohne sich gegenseitig zu beeinflussen. Mögliche Schaltungen zum Zusammenführen der unterschiedlichen Wechselspannungssignale können eine Parallelschaltung (Fig. 6c), eine Reihenschaltung (Fig. 6d) oder einer Mischung der beiden Schaltungen (Fig. 6e) darstellen.

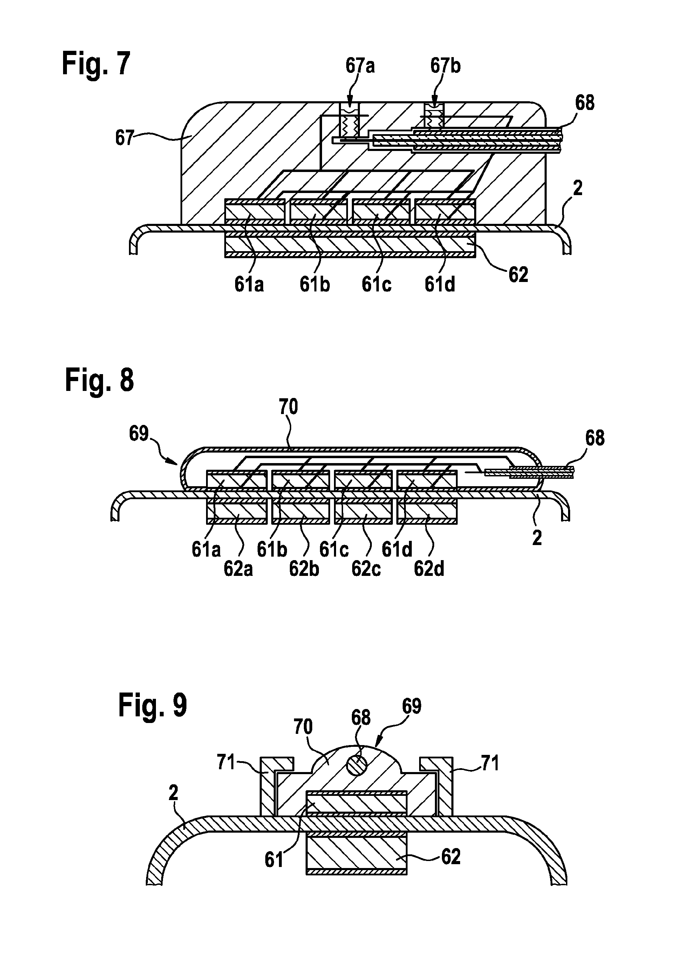

Fig. 7 stellt ein konstruktives Detail des mechanischen Abschnitts 6 dar, in dem eine Abdeckung 67 außerhalb der Außenwandung 2 angebracht ist. Die Abdeckung 67 deckt die außenliegenden Wandler 61a, 61b, 61c und 61d ab, um den außerhalb der Außenwandung 2 befindlichen mechanischen Abschnitt 6 zu schützen und um die Stabilität der Anordnung zu erhöhen. Innerhalb der Abdeckung 67 leiten die an die Kontaktflächen der Wandler 61 a, 61 b, 61 c und 61 d angeordneten elektrischen Leitungen die elektrische Signale zu einer Elektrode 68 weiter oder legen von dort kommende Signale an die Wandler an. Die Elektrodenleitung 68 ist an der Abdeckung 67 so angeschlossen, dass ihre Enden aus der Abdeckung 67 herausragen, um durch den elektrischen Abschnitt 5 mit dem Sensor 3 bzw. Aktor 4 in Signalverbindung zu stehen.

Als zusätzliche Anschlussmöglichkeit für den Sensor 3 bzw. Aktor 4 weist die Abdeckung 67 zwei Anschlüsse 67a und 67b auf, die mit der Elektrodenleitung 68 und den außerhalb der Außenwandung 2 befindlichen Wandler 61 a, 61 b, 61 c und 61 d in Verbindung stehen.

Der elektrische Anschluss eines Signalkabels erfolgt zweckmäßigerweise über genormte Verbindungen, wie zum Beispiel IS-1 oder IS-4 bzw. andere zukünftige, standardisierte Steckverbindungen, oder ein eigenständiges Steckersystem.

Fig. 8 zeigt eine wechselbare Elektrodenanschlussstruktur 69, die direkt an der Außenwandung 2 angebracht werden kann. Diese Struktur 69 weist ein Elektrodengehäuse 70 auf, an dem die piezoelektrischen Wandler 61 a, 61b, 61c und 61d befestigt sind. Die Wandler stehen mit der Außenwandung 2 in Kontakt, wodurch sie die mechanischen Schwingungen zu den innerhalb der Außenwandung 2 angeordneten zugehörigen Wandlern 62a, 62b, 62c und 62d übertragen können. Die Wandler 61a, 61 b, 61c und 61 d sind mit einer Elektrodenleitung 68 verbunden, welche ebenso am Elektrodengehäuse 70 befestigt ist. Ferner ragen die Enden der Elektrodenleitung 68 aus dem Elektrodengehäuse 70 heraus und stehen mit dem Sensor 3 und/oder Aktor 4 in Signalverbindung.

Die mechanische Befestigung dieser wechselbaren Elektrodenanschlussstruktur 69 kann auf verschiedene Arten erfolgen. In Fig. 9 ist eine Elektrodenanschlussstruktur 69 dargestellt, die in an der Außenwandung 2 des Implantats befestigten Schienen 71 eingeschoben oder eingeschnappt ist.

Im Übrigen ist die Ausführung der Erfindung nicht auf die oben beschriebenen Beispiele und hervorgehobenen Aspekte beschränkt, sondern lediglich durch den Schutzbereich der anhängenden Patentansprüche.

## Patentansprüche

1. Implantierbare Anordnung, aufweisend
ein implantierbares Gerät (1), insbesondere ein elektromedizinisches Gerät, mit einer Außenwandung (2), und
einem implantierbaren Sensor (3) und/oder Aktor (4), welcher in Signalverbindung mit dem implantierbaren Gerät steht,
wobei der implantierbare Sensor und/oder Aktor im Gebrauchszustand außerhalb der Außenwandung angeordnet ist und
wobei die Signalverbindung zwischen dem Gerät und dem Sensor und/oder Aktor einen elektrischen und einen hierzu in Reihe angeordneten mechanischen Abschnitt (5, 6) hat, welcher von einer Signalübertragung mittels mechanischer Schwingungen durch die geschlossene Außenwandung hindurch gebildet ist, wobei in dem mechanischen Abschnitt mindestens ein piezoelektrischer Wandler (61, 61 a, 61 b, 61 c, 61 d, 62, 62a, 62b, 62c, 62d, 66) zur Wandlung elektrischer Signale in mechanische Schwingungen oder umgekehrt vorgesehen ist,
**dadurch gekennzeichnet, dass**
sich auf mindestens einer Seite der Außenwandung (2) mindestens ein piezoelektrischer Wandler (66) befindet, der mit einer ungleichmäßigen Dicke zur Verwendung von verschiedenen Eigenfrequenzen gebildet ist.

2. Anordnung nach Anspruch 1, **dadurch gekennzeichnet, dass** jeweils ein piezoelektrischer Wandler (61, 61 a, 61 b, 61 c, 61 d, 62, 62a, 62b, 62c, 62d, 66) auf der einen und der anderen Seite der Außenwandung (2) angeordnet ist.

3. Anordnung nach Anspruch 1 oder 2, **dadurch gekennzeichnet, dass** der piezoelektrische Wandler (61, 61 a, 61 b, 61c, 61d, 62, 62a, 62b, 62c, 62d, 66) starr mit der Außenwandung (2) verbunden ist derart, dass er diese direkt in Schwingung versetzt.

4. Anordnung nach einem der Ansprüche 1 bis 3, **dadurch gekennzeichnet, dass** ein Schwingkreis vorgesehen ist, wobei der piezoelektrische Wandler (61, 61 a, 61 b, 61 c, 61 d, 62, 62a, 62b, 62c, 62d, 66) als Kondensator wirkt, der die elektrische Eigenfrequenz des Schwingkreises mindestens mitbestimmt.

5. Anordnung nach einem der vorangehenden Ansprüche, **dadurch gekennzeichnet, dass** innerhalb oder außerhalb der Außenwandung eine Gruppe mehrerer piezoelektrischer Wandler (61a, 61b, 61c, 61d, 62a, 62b, 62c, 62d) nebeneinander angeordnet ist, die verschiedene Resonanzfrequenzen besitzen.

6. Anordnung nach Anspruch 5, **dadurch gekennzeichnet, dass** auf der den mehreren Wandlern (61, 61a, 61 b, 61c, 61d) gegenüberliegenden Seite der Außenwandung (2) ein weiterer einzelner Wandler (62, 66) angeordnet ist und der einzelne Wandler mit einer Wechselspannung oder Wechselschwingung der Resonanzfrequenz eines oder mehrerer Wandler der Gruppe angesteuert wird.

7. Anordnung nach Anspruch 1, **dadurch gekennzeichnet, dass** innerhalb und außerhalb der Außenwandung (2) mehrere piezoelektrische Wandler (61a, 61b, 61c, 61 d, 62a, 62b, 62c, 62d) nebeneinander angeordnet sind, die verschiedene Resonanzfrequenzen besitzen, um Wandlerpaare zu bilden, die in Resonanz zu einander stehen.

8. Anordnung nach einem der vorangehenden Ansprüche, **dadurch gekennzeichnet, dass** außerhalb der Außenwandung (2) mindestens eine austauschbare oder fest installierte Elektrode (68) vorgesehen ist, zu der die elektrischen Signale durch eine Elektrodenleitung übertragen werden.

9. Anordnung nach Anspruch 8, **dadurch gekennzeichnet, dass** die Elektrodenleitungen im Bereich einer Abdeckung (67) angeschlossen sind, die sich an der Außenwandung befindet (2), um die Stabilität des an der Außenwandung befindlichen mechanischen Abschnittes (6) zu erhöhen, und diesen zu schützen.

10. Anordnung nach einem der vorangehenden Ansprüche, **dadurch gekennzeichnet, dass** in dem mechanischen Abschnitt (6) mindestens ein Element (65) vorgesehen ist, das die mechanischen Schwingungen zu einem weit entfernt von der Außenwandung angeordneten Ort weiterleitet.

11. Anordnung nach einem der vorangehenden Ansprüche, **dadurch gekennzeichnet, dass** das implantierbare Gerät einen Herzstimulator, der implantierbarer Sensor ein Mittel zur Messung der Herzkontraktionen und der implantierbare Aktor ein Mittel zur Erzeugung von Stimulierungsimpulsen darstellt.

12. Anordnung nach einem der Ansprüche 1 bis 10, **dadurch gekennzeichnet, dass** das implantierbare Gerät ein Hörgerätimplantat und der Aktor ein Mittel zur Stimulation des Hörnervs darstellt.

13. Anordnung nach einem der Ansprüche 1 bis 10, **dadurch gekennzeichnet, dass** das implantierbare Gerät ein orthopädisches Implantat und der Sensor ein Mittel zur Messung einer physiologischen Größe, wie die Temperatur, der Druck oder einer Spannung, darstellt.

## Claims

1. An implantable arrangement, comprising
an implantable device (1), in particular a medical device, having an outer wall (2), and
an implantable sensor (3) and/or actuator (4), which is connected to the implantable device such that signals can be exchanged,
wherein the implantable sensor and/or actuator is arranged outside the outer wall when in use, and
wherein the signal connection between the device and the sensor and/or actuator has an electric portion and a mechanical portion arranged in series therewith (5, 6), which mechanical portion is formed by a signal transfer by means of mechanical vibrations through the closed outer wall, wherein a piezoelectric transducer (61, 61 a, 61b, 61c, 61 d, 62, 62a, 62b, 62c, 62d, 66) for converting electric signals into mechanical vibrations or vice versa is provided in the mechanical portion, **characterised in that**
at least one piezoelectric transducer (66) is located on at least one side of the outer wall (2) and is formed with a non-uniform thickness for use of different natural frequencies.

2. The arrangement according to Claim 1, **characterised in that** a piezoelectric transducer (61, 61a, 61b, 61c, 61d, 62, 62a, 62b, 62c, 62d, 66) is arranged on either side of the outer wall (2).

3. The arrangement according to Claim 1 or 2, **characterised in that** the piezoelectric transducer (61, 61 a, 61b, 61c, 61 d, 62, 62a, 62b, 62c, 62d, 66) is rigidly connected to the outer wall (2) in such a way that it sets this in vibration directly.

4. The arrangement according to one of Claims 1 to 3, **characterised in that** a resonant circuit is provided, wherein the piezoelectric transducer (61, 61a, 61b, 61c, 61d, 62, 62a, 62b, 62c, 62d, 66) acts as a capacitor, which at least has an influence on the natural electric frequency of the resonant circuit.

5. The arrangement according to one of the preceding claims, **characterised in that** a group of a plurality of piezoelectric transducers (61, 61a, 61b, 61c, 61d, 62, 62a, 62b, 62c, 62d, 66) having different resonance frequencies is arranged side by side inside or outside the outer wall.

6. The arrangement according to Claim 5, **characterised in that** a further individual transducer (62, 66) is arranged on the side of the outer wall (2) opposite the plurality of transducers (61, 61a, 61b, 61c, 61d) and the individual transducer is actuated with an alternating voltage or alternating vibration of the resonance frequency of one or more transducers in the group.

7. The arrangement according to Claim 1, **characterised in that** a plurality of piezoelectric transducers (61, 61a, 61b, 61c, 61d, 62, 62a, 62b, 62c, 62d) are arranged side by side inside and outside the outer wall (2) and have different resonance frequencies in order to form transducer pairs, which are in resonance to one another.

8. The arrangement according to one of the preceding claims, **characterised in that** at least one exchangeable or fixedly installed electrode (68) is provided outside the outer wall (2), the electric signals being transferred to said electrode by an electrode line.

9. The arrangement according to Claim 8, **characterised in that** the electrode lines are connected in the region of a cover (67), which is located on the outer wall (2) in order to increase the stability of the mechanical portion (6) located on the outer wall and in order to protect said mechanical portion.

10. The arrangement according to one of the preceding claims, **characterised in that** at least one element (65) is provided in the mechanical portion (6), said element forwarding the mechanical vibrations to a location arranged far away from the outer wall.

11. The arrangement according to one of the preceding claims, **characterised in that** the implantable device constitutes a heart stimulator, the implantable sensor constitutes a means for measuring the heart contractions, and the implantable actuator constitutes a means for producing stimulation pulses.

12. The arrangement according to one of Claims 1 to 10, **characterised in that** the implantable device constitutes a hearing device implant and the actuator constitutes a means for stimulation of the auditory nerve.

13. The arrangement according to one of Claims 1 to 10, **characterised in that** the implantable device constitutes an orthopaedic implant and the sensor constitutes a means for measuring a physiological variable, such as the temperature, the pressure or a voltage.

## Revendications

1. Dispositif implantable comportant
un appareil implantable (1), en particulier un appareil électromédical, avec une paroi extérieure (2), et
un capteur (3) et/ou un acteur (4) implantable, relié par signaux à l'appareil implantable,
dans lequel le capteur et/ou l'acteur implantable est agencé à l'extérieur de la paroi extérieure à l'état de service, et
dans lequel la liaison par signaux entre l'appareil et le capteur et/ou l'acteur comporte une section électrique et une section mécanique (5, 6) agencée en série par rapport à celle-ci, laquelle est formée par une transmission de signaux au moyen d'oscillations mécaniques à travers la paroi extérieure fermée, dans lequel il est prévu au moins un convertisseur piézoélectrique (61, 61a, 61b, 61c, 61d, 62, 62a, 62b, 62c, 62d, 66) dans la section mécanique, pour la conversion des signaux électriques en oscillations mécaniques ou inversement,
**caractérisé en ce que**
au moins un convertisseur piézoélectrique (66) formé avec une épaisseur irrégulière pour l'utilisation de différentes fréquences propres se trouve au moins d'un côté de la paroi extérieure (2).

2. Dispositif selon la revendication 1, **caractérisé en ce qu'**un convertisseur piézoélectrique (61, 61a, 61b, 61c, 61d, 62, 62a, 62b, 62c, 62d, 66) est agencé respectivement d'un côté et de l'autre de la paroi extérieure (2).

3. Dispositif selon la revendication 1 ou 2, **caractérisé en ce que** le convertisseur piézoélectrique (61, 61a, 61b, 61c, 61d, 62, 62a, 62b, 62c, 62d, 66) est relié fixement à la paroi extérieure (2), de manière à la faire osciller directement.

4. Dispositif selon l'une des revendications 1 à 3, **caractérisé en ce qu'**il est prévu un circuit oscillant, le convertisseur piézoélectrique (61, 61a, 61b, 61c, 61d, 62, 62a, 62b, 62c, 62d, 66) agissant comme un condensateur participant à la détermination de la fréquence électrique propre du circuit oscillant.

5. Dispositif selon l'une des revendications précédentes, **caractérisé en ce qu'**un groupe de plusieurs convertisseurs piézoélectrique (61, 61a, 61b, 61c, 61d, 62, 62a, 62b, 62c, 62d, 66) agencés côte à côte et présentant différentes fréquences de résonnance est prévu à l'intérieur ou à l'extérieur de la paroi extérieure.

6. Dispositif selon la revendication 5,
**caractérisé en ce que** du côté de la paroi extérieure (2) opposé aux plusieurs convertisseurs piézoélectrique (61, 61a, 61b, 61c, 61d), il est prévu un convertisseur piézoélectrique (62, 66) individuel supplémentaire, et le convertisseur supplémentaire est actionné avec une tension alternative ou une oscillation alternative de la fréquence de résonnance d'un ou plusieurs convertisseurs du groupe.

7. Dispositif selon la revendication 1, **caractérisé en ce qu'**il est prévu plusieurs convertisseur piézoélectrique (61, 61a, 61b, 61c, 61d, 62, 62a, 62b, 62c, 62d) agencés côte à côte à l'intérieur et à l'extérieur de la paroi extérieure (2), lesquels présentent différentes fréquences de résonnance, pour former des paires de convertisseurs en résonnance les uns avec les autres.

8. Dispositif selon l'une des revendications précédentes, **caractérisé en ce qu'**à l'extérieur de la paroi extérieure (2), il est prévu au moins une électrode (68) installée fixement ou remplaçable, vers laquelle sont transmis les signaux électriques par le biais d'une ligne d'électrodes.

9. Dispositif selon la revendication 8, **caractérisé en ce que** les lignes d'électrodes sont raccordées dans la région d'un recouvrement (67) situé sur la paroi extérieure (2), pour augmenter la stabilité de la section mécanique (6) située sur la paroi extérieure et protéger celle-ci.

10. Dispositif selon l'une des revendications précédentes, **caractérisé en ce que** dans la section mécanique (6), il est prévu au moins un élément (65) transmettant les oscillations mécaniques vers un emplacement très éloigné de la paroi extérieure.

11. Dispositif selon l'une des revendications précédentes, **caractérisé en ce que** l'appareil implantable représente un stimulateur cardiaque, le capteur implantable représente un moyen de mesure de contractions cardiaques et l'acteur implantable représente un moyen de génération d'impulsions de stimulation.

12. Dispositif selon l'une des revendications 1 à 10, **caractérisé en ce que** l'appareil implantable représente un implant d'appareil auditif et l'acteur représente un moyen de stimulation du nerf auditif.

13. Dispositif selon l'une des revendications 1 à 10, **caractérisé en ce que** l'appareil implantable représente un implant orthopédique et le capteur représente un moyen de mesure d'une valeur physiologique, telle que la température, la pression ou la tension.
